# EUROPEAN PATENT APPLICATION

(11) **EP 3 520 771 A1**
(43) Date of publication of application: **07.08.2019**
(21) Application number: 17856687.3
(22) Date of filing: 26.09.2017
(51) Int. Cl.: A61K 8/49, A23L 33/10, A61Q 19/00

(54) **ANTI-STRESS COMPOSITION CONTAINING COSMOPERINE**

(30) Priority: 27.09.2016 KR 20160124253
(71) Applicant: Amorepacific Corporation, Seoul 04386 (KR)
(72) Inventor: KIM, Juewon, Yongin-si Gyeonggi-do 17074 (KR); CHOI, Minsik, Yongin-si Gyeonggi-do 17074 (KR); HONG, Yong Deog, Yongin-si Gyeonggi-do 17074 (KR); CHO, Siyoung, Yongin-si Gyeonggi-do 17074 (KR)
(74) Representative: Novagraaf Technologies
(86) International application number: PCT/KR2017/010586
(87) International publication number: WO 2018/062801

(57) **Abstract**

The present specification describes an anti-stress composition containing, as an active ingredient, cosmoperine, an isomer thereof, a pharmaceutically or cosmetically acceptable salt thereof, a hydrate thereof or a solvate thereof. The composition can alleviate or relieve physical and psychological symptoms caused by stress.

## Description

### [Technical Field]

The present specification describes an anti-stress composition comprising cosmoperine, an isomer thereof, a pharmaceutically or cosmetically acceptable salt thereof, a hydrate thereof or a solvate thereof.

### [Background Art]

Stress, which indicates the maladaptation of the human body due to external stimuli or environmental changes, can lead to hyperactivity of the sympathetic nervous system, which may result in temporary or continuous psychological and physical responses. Modern people are exposed to various stress sources, and these are closely related to various changes in the state of mind and body. Thus, there is a growing interest in resistance to stress.

Therapeutic agents for depression and stress using a synthetic compound may cause side effects or human toxicity. There is a need for a substance having an anti-stress effect as a cosmetic or food, rather than as a drug, and which uses a natural product.

### [Summary of Invention]

### [Technical Problem]

In one aspect, an object of the present invention is to provide a composition having the effect of improving resistance to stress.

In another aspect, an object of the present invention is to provide a composition having the effect of improving resistance to stress without causing human toxicity by using a natural product.

In another aspect, an object of the present invention is to provide a composition having an excellent resistance to glucocorticoid hormone-induced stress.

In another aspect, an object of the present invention is to provide a composition capable of improving resistance to stress through activation of the oxytocin receptor.

In another aspect, an object of the present invention is to provide a composition that alleviates or relieves physical symptoms caused by stress.

In another aspect, an object of the present invention is to provide a composition that alleviates or relieves psychological symptoms caused by stress.

### [Solution to Problem]

In one aspect, the present invention provides an anti-stress composition comprising, as an active ingredient, cosmoperine, an isomer thereof, a pharmaceutically or cosmetically acceptable salt thereof, a hydrate thereof or a solvate thereof.

### [Advantageous Effects of Invention]

In one aspect, the present invention achieves the effect of improving resistance to stress.

In another aspect, the present invention achieves the effect of improving resistance to stress without causing human toxicity by using a natural product.

In another aspect, the present invention allows to achieve an excellent resistance to glucocorticoid hormone-induced stress.

In another aspect, the present invention allows to improve resistance to stress through activation of the oxytocin receptor.

In another aspect, the present invention can alleviate or relieve physical symptoms caused by stress.

In another aspect, the present invention can alleviate or relieve psychological symptoms caused by stress.

### [Brief Description of Drawings]

FIG. 1 is a graph showing the binding of cosmoperine to the oxytocin receptor.
FIG. 2 is a graph showing the effect of cosmoperine on the inhibition of glucocorticoid-induced apoptosis.

### [Description of Embodiments]

### [Embodiments]

Hereinafter, embodiments of the present invention will be described in detail with reference to the accompanying drawings. However, the technology disclosed in the present invention is not limited to the embodiments set forth herein and may be embodied in a variety of different forms. Rather, these embodiments are provided so that this disclosure will be thorough and complete and fully convey the spirit of the invention to those skilled in the art. The width, thickness, etc. of the elements in the drawings are slightly exaggerated in order to clearly illustrate each element. In addition, only part of the elements may be illustrated for convenience of explanation. However, those skilled in the art could easily conceive the rest of the elements. Also, those skilled in the art could embody the spirit of the present invention in various other forms without departing from the spirit of the invention.

As used herein, the "isomer" comprises not only optical isomers (e.g., essentially pure enantiomers, essentially pure diastereomers or a mixture thereof) but also conformational isomers (i.e., isomers in which only the angle of one or more chemical bonds is different between each other), positional isomers (particularly, tautomers) or geometric isomers (e.g., cis-trans isomers).

As used herein, "essentially pure" means, when used, for example, with regard to enantiomers or diastereomers, that specific compounds, e.g., enantiomers or diastereomers, are present in an amount of about 90% (w/w) or more, preferably about 95% or more, more preferably about 97% or more or about 98% or more, more preferably about 99% or more, even more preferably about 99.5% or more.

As used herein, "pharmaceutically acceptable" means being devoid of substantial toxic effects when used in a usually employed medicinal dosage and thereby being approvable or approved by the government or a regulatory organization comparable thereto or being listed in the Pharmacopeia or recognized by other general pharmacopoeias for use in animals, and more particularly in humans.

As used herein, the "pharmaceutically or cosmetically acceptable salt" refers to a salt according to one aspect of the present invention that is pharmaceutically or cosmetically acceptable and possesses the desired pharmacological activity of the parent compound. Such salts include: (1) acid addition salts formed from an inorganic acid such as hydrochloric acid, hydrobromic acid, sulfuric acid, nitric acid, phosphoric acid, etc., or formed from an organic acid such as acetic acid, propionic acid, hexanoic acid, cyclopentane propionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid and muconic acid; or (2) salts formed when an acidic proton present in the parent compound is substituted.

As used herein, the "hydrate" refers to a compound to which water is bonded. The term is used in a broad sense, including an inclusion compound which lacks chemical bonding with water.

As used herein, the "solvate" refers to a high order compound formed between a molecule or ion of solute, and a molecule or ion of solvent.

According to one embodiment, the present invention may provide an anti-stress composition comprising, as an active ingredient, cosmoperine, an isomer thereof, a pharmaceutically or cosmetically acceptable salt thereof, a hydrate thereof or a solvate thereof.

In this embodiment, cosmoperine, also referred to as tetrahydropiperine, is a piperine-based compound having a structure represented by the formula below. Cosmoperine is known as a substance that facilitates permeation of a water-soluble substance. The present inventors have found that cosmoperine has the effect of improving resistance to stress. Therefore, the composition according to this embodiment can exhibit an excellent effect as an anti-stress composition.

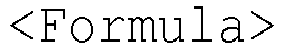

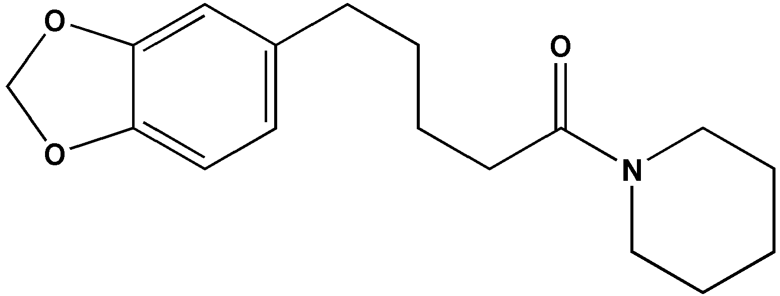

The composition is resistant to glucocorticoid hormone-induced stress. In one embodiment, the composition may inhibit glucocorticoid hormone-induced apoptosis. The inhibition of glucocorticoid hormone-induced apoptosis may result in the alleviation or relief of psychological or physical symptoms. The glucocorticoid hormone may include at least one selected from cortisol, cortisone and corticosterone.

The composition can bind to the oxytocin receptor to activate the oxytocin receptor. Oxytocin (OXT, OT) is a neurotransmitter secreted from the posterior pituitary of various animals, including vertebrates and invertebrates. It is also referred to as the uterine contraction hormone. It is known that oxytocin is involved in contraction of the uterus during childbirth and is also secreted into the bloodstream from the pituitary gland when one sees a charming person. The effects of oxytocin on stress relief and sociality have also been reported. The composition according to this embodiment can achieve resistance to stress by activating the oxytocin receptor.

The composition can alleviate or relieve physical symptoms caused by stress. Examples of the physical symptoms caused by stress include symptoms of digestive symptoms such as irritable colitis, indigestion, gastritis, stomach cramps, gastroduodenal ulcer, and constipation, neurological symptoms such as migraine, urinary symptoms such as erectile dysfunction and sex frigidity, cardiovascular symptoms such as hypertension and angina, and skin symptoms such as hair loss and urticaria. Persistent stress may make these symptoms develop into diseases.

The composition can alleviate or relieve psychological symptoms caused by stress. Examples of the psychological symptoms caused by stress include depression, anxiety disorder, insomnia and nervousness.

In one embodiment, the composition may be a cosmetic composition.

The cosmetic composition according to one embodiment of the present invention may include 0.0001% by weight to 99% by weight, for example, 0.01% by weight to 60% by weight of the active ingredient based on the total weight of the composition, although not limited thereto.

The cosmetic composition according to one embodiment of the present invention may be formulated to contain a cosmetically or dermatologically acceptable medium or base. It may be prepared into any formulation suitable for topical application, for example, a suspension, a microemulsion, a microcapsule, a microgranule, an ionic (liposome) or nonionic vesicle dispersant, a cream, a skin lotion, a nourishing lotion, a powder, an ointment, a spray or a conceal stick. Also, it may be used in the form of a foam or an aerosol composition further containing a compressed propellant. The composition may be prepared according to a method commonly employed in the art.

Further, the cosmetic composition according to the embodiments of the present invention may include an adjuvant conventionally used in the field of cosmetics or dermatology, such as a powder, fat, an organic solvent, a dissolving agent, a concentrating agent, a gelling agent, a softener, an antioxidant, a suspending agent, a stabilizer, a foaming agent, a fragrance, a surfactant, water, an ionic or nonionic emulsifier, a filler, a metal ion blocker, a chelating agent, a preservative, vitamins, a blocking agent, a wetting agent, essential oil, a dye, a pigment, a hydrophilic or hydrophobic activating agent, lipid vesicles or other ingredients conventionally used in cosmetics. The adjuvant may be used in an amount conventionally used in the field of cosmetics or dermatology. The cosmetic composition according to the embodiments of the present invention may further contain a skin absorption promoting substance to increase the effect of improving the skin.

The composition according to the embodiments of the present invention may be provided as various types of food additives or functional foods. For example, it may be processed into fermented milk, cheese, yogurt, juice, probiotics, health food, etc. containing the active ingredient, as well as various food additives.

The composition according to the embodiments of the present invention may be a composition for health foods.

The composition for health foods according to one embodiment of the present invention may include 0.0001% by weight to 99% by weight, for example, 0.01% by weight to 60% by weight of the active ingredient based on the total weight of the composition, although not limited thereto.

In one specific embodiment, the composition for health foods may be formulated into a pill, a capsule, a tablet, a granule, a caramel or a drink. In other specific embodiments, it may be processed into the form of a liquid, a powder, a granule, a tablet, a tea bag or the like.

The composition may be administered by various methods such as drinking, injection, spraying or squeezing.

The composition may contain other ingredients that can provide synergic effects without negatively affecting the main effect of the present invention. For example, in order to improve the properties, it may further include an additive such as flavoring agents, a pigment, a sterilizer, an antioxidant, a preservative, a humectant, a thickener, a mineral salt, an emulsifier, a synthetic polymer, etc. In addition, it may further include an auxiliary ingredient such as a water-soluble vitamin, an oil-soluble vitamin, a polypeptide, a polysaccharide, seaweed extract, etc. These ingredients may be appropriately selected and combined by those skilled in the art considering the formulation or use. The amount of these ingredients may be determined within a range not negatively affecting the object and effect of the present invention. For example, the amount of the ingredients may be 0.01% by weight to 5% by weight, for example, 0.01% by weight to 3% by weight based on the total weight of the composition, although not limited thereto.

### [Examples]

Hereinafter, the present invention will be described in further detail with reference to examples. It will be apparent to those skilled in the art that these examples are for illustrative purposes only, and the present invention should not be construed as limited to the examples set forth herein.

### Test Example 1: Confirmation of binding to the oxytocin receptor

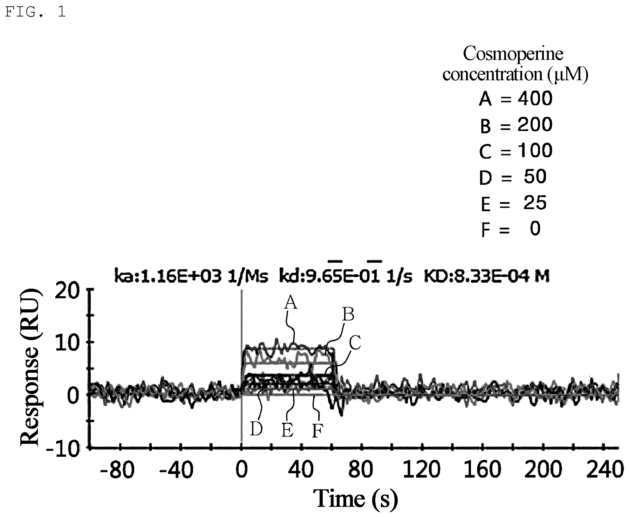

In order to confirm the binding of cosmoperine to the oxytocin receptor, cosmoperine (Sabina Corp, NJ) at a concentration of 0 to 400 µM was applied to a SPR chip (Biorad, MA) with the oxytocin receptor (oxytocin receptor recombinant protein, Mybiosource, MA) bound to its surface, followed by reaction for 90 seconds. Then, the binding pattern of cosmoperine to the oxytocin receptor was measured using a surface plasmon resonance device (Surface plasmon resonance; Biorad, MA). The results are shown in FIG. 1.

From the results of FIG. 1, it was found that cosmoperine binds to the oxytocin receptor in a concentration-dependent manner.

### Test Example 2: Inhibitory effect on glucocorticoid-induced apoptosis

In order to confirm the anti-stress effect of cosmoperine, a test was performed for glucocorticoid-induced apoptosis.

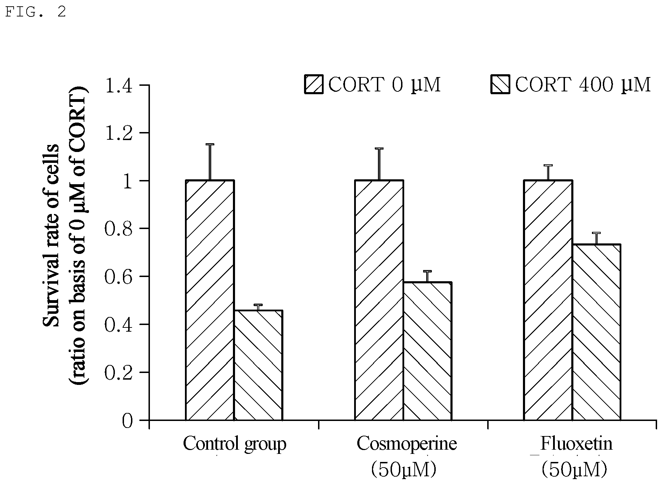

PC12 (neurocytoma cell; KCLB21721, Korea Cell Line Bank) was seeded into a 96-well plate at 2x10⁴ cells/well and cultured at 5% CO₂ and 37°C. The culture medium was RPMI 1640 medium (Gibco BRL, Grand Island, NY, USA)) supplemented with 10% serum (heat-inactivated fetal bovine serum (FBS, Gibco)), 100 U/ml of penicillin (Gibco) and 100 µg/ml of streptomycin (Gibco). The cells were treated with 400 µM corticosterone (Sigma, MA), which is a stress hormone, and cultured at 5% CO₂ and 37°C for 24 hours to induce stress. 400 µM corticosterone and 50 µM fluoxetine (fluoxetine hydrochloride, Sigma), which is an antidepressant, were used as the positive control group. For the cosmoperine-treated group, 400 µM corticosterone and 50 µM cosmoperine (Sabina Corp, NJ) were used. Fluoxetine and cosmoperine each were pretreated 2 hours before the application of corticosterone. 10 µl of CCK-8 solution (Dojindo Laboratories) was added to each of the wells, followed by culture for 2 hours. Then, measurements were made at 540nm. Based on the measurement results, FIG. 2 shows the relative survival rate of cells in the case where the value of the group not treated with corticosterone is 1.

From the results of FIG. 2, it can be seen that cosmoperine is excellent in inhibiting the apoptosis induced by corticosterone, which is a representative stress-related glucocorticoid hormone.

### Formulation Example 1: Tablet

100 mg of the eluate of Preparation Example 1 of the present invention, 400 mg of lactose, 400 mg of corn starch and 2 mg of magnesium stearate were mixed and tableted according to a conventional method for preparing tablets to prepare a tablet.

### Formulation Example 2: Capsule

100 mg of the eluate of Preparation Example 1 of the present invention, 400 mg of lactose, 400 mg of corn starch and 2 mg of magnesium stearate were mixed and filled in a gelatin capsule according to a conventional method for preparing capsules to prepare a capsule.

### Formulation Example 3: Granule

50 mg of the eluate of Preparation Example 1 of the present invention, 250 mg of anhydrous crystalline glucose and 550 mg of starch were mixed and granulated into granules using a fluidized bed granulator, which were then filled in a pouch.

### Formulation Example 4: Drink

50 mg of the eluate of Preparation Example 1 of the present invention, 10 g of glucose, 0.6 g of citric acid and 25 g of liquid oligosaccharide were mixed and added with 300 ml of purified water. 200 ml of the resultant mixture was filled into each bottle. After filled into a bottle, the mixture was sterilized at 130°C for 4 to 5 seconds to prepare a drink.

### Formulation Example 5: Softening lotion (skin lotion)

A softening lotion was prepared according to a conventional method with the composition shown in the table below.

**Table 1**

| Ingredient | Content (% by weight) |
|---|---|
| Cosmoperine | 0.2 |
| Glycerin | 3.0 |
| Butylene glycol | 2.0 |
| Propylene glycol | 2.0 |
| Carboxyvinyl polymer | 0.1 |
| PEG-12 nonylphenyl ether | 0.2 |
| Polysorbate 80 | 0.4 |
| Ethanol | 10.0 |
| Triethanolamine | 0.1 |
| Preservative, colorant, fragrance | q.s. |
| Purified water | To 100 |

### Formulation Example 6: Nourishing lotion (milk lotion)

A nourishing lotion was prepared according to a conventional method with the composition shown in the table below.

**Table 2**

| Ingredient | Content (% by weight) |
|---|---|
| Cosmoperine | 1.0 |
| Glycerin | 3.0 |
| Butylene glycol | 3.0 |
| Propylene glycol | 3.0 |
| Carboxyvinyl polymer | 0.1 |
| Wax | 4.0 |
| Polysorbate 60 | 1.5 |
| Caprylic/capric triglyceride | 5.0 |
| Squalane | 5.0 |
| Sorbitan sesquioleate | 1.5 |
| Liquid paraffin | 0.5 |
| Cetearyl alcohol | 1.0 |
| Triethanolamine | 0.2 |
| Preservative, colorant, fragrance | q.s. |
| Purified water | To 100 |

### Formulation Example 7: Massage cream

A massage cream was prepared according to a conventional method with the composition shown in the table below.

**Table 3**

| Ingredient | Content (% by weight) |
|---|---|
| Cosmoperine | 2.0 |
| Glycerin | 8.0 |
| Butylene glycol | 4.0 |
| Liquid paraffin | 45.0 |
| Beta-glucan | 7.0 |
| Carbomer | 0.1 |
| Caprylic/capric triglyceride | 3.0 |
| Wax | 4.0 |
| Cetearyl glucoside | 1.5 |
| Sorbitan sesquioleate | 0.9 |
| Vaseline | 3.0 |
| Paraffin | 1.5 |
| Preservative, colorant, fragrance | q.s. |
| Purified water | To 100 |

### Formulation Example 8: Preparation of hair lotion

A hair lotion was prepared according to a conventional method with the composition shown in the table below.

**Table 4**

| Ingredient | Content (% by weight) |
|---|---|
| Cosmoperine | 2.0 |
| Purified water | To 100 |
| Glycerin | 3.0 |
| Butylene glycol | 3.0 |
| Liquid paraffin | 7.0 |
| Beta-glucan | 7.0 |
| Carbomer | 0.1 |
| Edelweiss extract | 2.0 |
| Caprylic/capric triglyceride | 3.0 |
| Squalene | 5.0 |
| Cetearyl glucoside | 1.5 |
| Sorbitan stearate | 0.4 |
| Polysorbate | 1.2 |
| Preservative | q.s. |
| Fragrance | q.s. |
| Colorant | q.s. |
| Triethanolamine | 0.1 |

### Formulation Example 9: Preparation of shampoo composition

A shampoo composition was prepared according to a conventional method with the composition shown in the table below.

**Table 5**

| Ingredient | Content (% by weight) |
|---|---|
| Polyquaternium-10 | 0.5 |
| Sodium laureth sulfate | 20 |
| Cosmoperine | 0.3 |
| Fragrance | 1.0 |
| Methyl paraben | 0.1 |
| Cetyl alcohol | 0.5 |
| Sodium chloride | 0.8 |
| Purified water | To 100 |

### Formulation Example 10: Preparation of hair treatment

A hair treatment was prepared according to a conventional method with the composition shown in the table below.

**Table 6**

| Ingredient | Content (% by weight) |
|---|---|
| Cosmoperine | 0.5 |
| Stearamidopropyl dimethylamine | 2 |
| Glycerin | 0.5 |
| Fragrance | 0.5 |
| Preservative | 0.03 |
| Cetostearyl alcohol | 5 |
| Lactic acid | 1 |
| Distilled water | To 100 |

## Claims

1. An anti-stress composition comprising, as an active ingredient, cosmoperine, an isomer thereof, a pharmaceutically or cosmetically acceptable salt thereof, a hydrate thereof or a solvate thereof.

2. The composition according to claim 1,
wherein cosmoperine is a compound having a structure represented by the following formula:

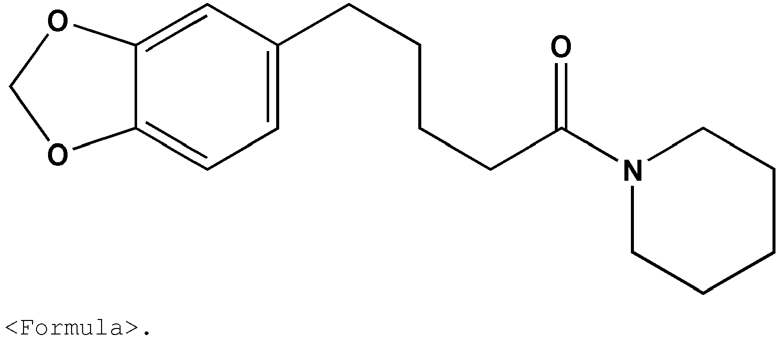

3. The composition according to claim 1,
wherein the composition exhibits resistance to glucocorticoid hormone-induced stress.

4. The composition according to claim 3,
wherein the glucocorticoid hormone comprises at least one selected from cortisol, cortisone and corticosterone.

5. The composition according to claim 1,
wherein the composition alleviates or relieves physical symptoms caused by stress.

6. The composition according to claim 1,
wherein the composition alleviates or relieves psychological symptoms caused by stress.

7. The composition according to claim 1,
wherein the composition is a cosmetic composition.

8. The composition according to claim 7,
wherein the content of the active ingredient is 0.01% by weight to 99% by weight based on the total weight of the composition.

9. The composition according to claim 1,
wherein the composition is a composition for health foods.

10. The composition according to claim 9,
wherein the content of the active ingredient is 0.01% by weight to 99% by weight based on the total weight of the composition.
